(19) Europäisches Patentamt
European Patent Office
Office européen des brevets

(11) **EP 3 381 938 B1**

(12) **EUROPEAN PATENT SPECIFICATION**

(45) Date of publication and mention
of the grant of the patent:
**03.02.2021 Bulletin 2021/05**

(51) Int Cl.:
***C07K 14/78*** *(2006.01)* ***C12N 13/00*** *(2006.01)*

(21) Application number: **15909357.4**

(22) Date of filing: **01.12.2015**

(86) International application number:
**PCT/KR2015/012988**

(87) International publication number:
**WO 2017/090808 (01.06.2017 Gazette 2017/22)**

(54) **METHOD FOR INCREASING COLLAGEN YIELD, AND COLLAGEN PREPARED USING SAME**

VERFAHREN ZUR ERHÖHUNG DER KOLLAGENAUSBEUTE UND DAMIT HERGESTELLTES KOLLAGEN

PROCÉDÉ D'AUGMENTATION DU RENDEMENT DU COLLAGÈNE ET COLLAGÈNE PRÉPARÉ AVEC CE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.11.2015 KR 20150164277**

(43) Date of publication of application:
**03.10.2018 Bulletin 2018/40**

(73) Proprietor: **Sewoncellontec Co., Ltd.**
**Seoul 07325 (KR)**

(72) Inventors:
• **LEE, Joon Ho**
**Seoul 05513 (KR)**
• **YOO, Ji Chul**
**Namyangju-si**
**Gyeonggi-do 12010 (KR)**
• **JEONG, Hyeong Woo**
**Goyang-si**
**Gyeonggi-do 10504 (KR)**
• **CHOI, Sang Bum**
**Seoul 02049 (KR)**
• **SUH, Dong Sam**
**Seoul 01742 (KR)**
• **CHANG, Cheong Ho**
**Seoul 06195 (KR)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) References cited:
**WO-A1-2006/096027      WO-A1-2011/014155**
**WO-A2-2006/124988      KR-A- 20110 068 948**
**KR-A- 20140 118 753      KR-A- 20150 102 865**
**KR-B1- 100 676 285      KR-B1- 100 837 858**

## Description

## Technical Field

Embodiments of the present invention relate to a method of increasing a collagen yield and collagen prepared thereby, and more particularly to a method of increasing a collagen yield and collagen prepared thereby, in which animal-derived tissue is irradiated with radioactive rays to thus remove microorganisms and endotoxins therefrom, thereby increasing preservability and the yield of collagen without collagen denaturation, whereby the quality and reliability of collagen may be drastically improved to thus satisfy diverse needs of users, ultimately realizing improved product viability.

## Background Art

As is well known in the art, collagen is a light protein that constitutes the tissues of skin, bones and cartilage of various animals, including mammals and birds, as well as fish scales, and is currently known to comprise about 20 kinds thereof, Type I collagen constituting most such tissues.

Collagen may be obtained from tissues of animals through extraction and purification processes using enzyme separation, organic solvent separation, acid/alkali/neutral separation, or the like.

Although methods for increasing the yield of collagen have been studied, it is currently difficult to increase the yield without increasing the separation processing time or mixing certain chemicals.

Collagen is currently industrially processed in various forms in the fields of medicine, food, cosmetics, and the like. In particular, since collagen is excellent in biocompatibility and tissue affinity and is absorbed after complete degradation *in vivo*, it is receiving a great attention in the field of medicine.

In order to use collagen for medical purposes, resistance to various biological contaminants including viruses and bacteria has to be ensured.

Sterilization methods for removing contaminants include heat treatment (wet and dry), chemical solvent treatment, and treatment with radioactive rays.

The above methods are problematic in that collagen may be denatured into a form unsuitable for the purpose of use due to the generation of residue that may be toxic or changes in the properties of materials to be sterilized.

With the goal of solving the above problems, the following patent documents have been developed, but there still remains an important issue in that the problems of the related art described above cannot be solved at all.

[Citation List]

(Patent Document 1) Korean Patent No. 0837858 (Registration Date: June 05, 2008)
(Patent Document 2) Korean Patent Application Publication No. 2012-0084189 (Laid-open Date: July 27, 2012)
(Patent Document 3) Korean Patent Application Publication No. 2014-0118763 (Laid-open Date: October 08, 2014)

WO 2011/014155 discloses a method for producing a collagen preparation, the method comprising providing a connective tissue having a surface ranging from 20 $mm^2$ to 2 $m^2$, swelling the connective tissue with a first acidic solution by at least 50% in volume to form a swollen connective tissue, wherein the acidic solution is substantially free of salt and has ha pH of 1-6, washing the swollen connective tissue to remove non-collagenous material, thereby producing a collagen matrix, and extracting collagen from the collagen matrix with an extraction solution to produce a collagen-containing solution.

WO 2006/096027 refers to a method for separation of collagen from various animal tissues, comprising, upon final treatment of collagen, treating a collagen solution having a predetermined concentration under neutral conditions at a low temperature, followed by overnight treatment at a temperature of 30 to 35 °C, concentrating collagen by centrifugation, and dissolving the thus-concentrated collagen in refrigerated weakly-acidic solvent or phosphate buffered saline (PBS), thereby preparing a solution having a collagen concentration of 1 to 5 mg/ml.

WO 2006/124988 is concerned with a method for sterilizing collagen material or dermal filler comprising: (a) freezing the collagen material or dermal filler and (b) irradiating the collagen material or dermal filler with an effective amount of gamma or e-beam radiation to sterilize the collagen material or dermal filler without causing significant deterioration of the collagen material or dermal filler.

US 2016/0052962 relates to a method for separating collagen from jellyfish by using radiation, the method comprising the following steps: i) washing and pulverizing the jellyfish; ii) dipping the pulverized jellyfish prepared in step i) in an acid solution; iii) irradiating the solution of step ii), followed by stirring, and iv) filtering the stirred solution of step iii) and drying thereof.

**Disclosure**

**Technical Problem**

Accordingly, the present invention has been made keeping in mind the above problems encountered in the related art, and the first object of the present invention is to increase the yield of collagen by sterilizing animal-derived tissue through irradiation with radioactive rays, followed by washing, pulverization, separation, extraction and desalting. The second object of the present invention is to increase preservability by irradiating animal-derived tissue with radioactive rays to thus remove microorganisms and endotoxins therefrom. The third object of the present invention is to increase the yield of collagen without collagen denaturation. The fourth object of the present invention is to generate economic benefits by simplifying industrial processing and reducing unnecessary energy consumption because the sterilization of tissue and an increase in the yield of collagen may be realized through a single process. The fifth object of the present invention is to sterilize animal-derived tissue and increase the yield of collagen using radioactive rays, which are environmentally friendly and have high industrial applicability. The sixth object of the present invention is to provide a method of increasing a collagen yield and collagen prepared thereby, in which the quality and reliability of collagen may be drastically improved to thus satisfy diverse needs of users, ultimately realizing improved product viability.

**Technical Solution**

In order to accomplish the above objects, the present invention provides a method of increasing a collagen yield, suitable for sterilizing animal-derived tissue through irradiation with radioactive rays without collagen denaturation and simultaneously increasing the yield of collagen, the method comprising: sterilizing animal-derived tissue through irradiation with radioactive rays in dose of 5 kGy ~ 40 kGy; washing and pulverizing the sterilized animal-derived tissue; separating collagen by reacting the pulverized tissue with an acid solution or an enzyme solution, the stirring time being 3 ~ 7 days in case an acid solution is used; extracting collagen through addition of a salt; and removing the salt from the separated collagen.

**Advantageous Effects**

As described hereinbefore, according to the present invention, the yield of collagen can be increased by sterilizing animal-derived tissue through irradiation with radioactive rays, followed by washing, pulverization, separation, extraction and desalting.
According to the present invention based on the above technical construction, preservability can be increased by irradiating animal-derived tissue with radioactive rays to thus remove microorganisms and endotoxins therefrom.
According to the present invention, the yield of collagen can be increased without denaturation thereof.
According to the present invention, the sterilization of tissue and an increase in the yield of collagen can be realized through a single process, thus simplifying industrial processing and reducing unnecessary energy consumption to thereby generate economic benefits.
Furthermore, animal-derived tissue can be sterilized and the yield of collagen can be increased using radioactive rays, which are environmentally friendly and have high industrial applicability.
Because of the above effects, the quality and reliability of collagen can be drastically improved to thus satisfy diverse needs of users, ultimately realizing improved product viability.
Preferred embodiments of the present invention for achieving the above effects are described in detail below, with reference to the accompanying drawings.

**Description of Drawings**

FIG. 1 shows the process of separating collagen from animal-derived tissue sterilized through irradiation with radioactive rays according to an embodiment of the present invention;
FIG. 2 is a photograph showing animal-derived tissue irradiated with gamma rays after sealing in order to realize long-term preservation according to the present invention;
FIG. 3 shows the extent of sterilization of bacteria and fungi after irradiation of pig skin tissue with gamma rays according to the present invention;
FIG. 4 shows the extent of sterilization of bacteria and fungi after irradiation of skin tissue of duck's feet with gamma rays according to the present invention;
FIG. 5 is a graph showing the yield of collagen separated through acid treatment with gamma rays applied in different amounts according to the present invention;
FIG. 6 is of photographs showing collagen lyophilized after separation through acid treatment with gamma rays applied in different amounts according to the present invention; and
FIG. 7 is a photograph showing the results of SDS-PAGE of collagen separated through acid treatment with gamma rays applied in different amounts according to the present invention.

**Best Mode**

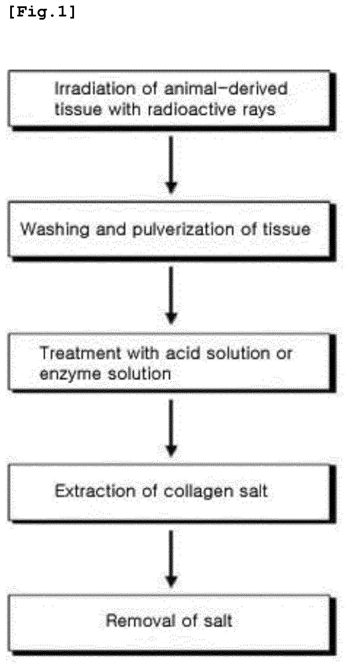

The construction of the present invention is provided as shown in FIGS. 1 to 7.

In the following description of the present invention, it is to be noted that a detailed description of related known functions or constructions will be omitted when it would make the gist of the present disclosure unclear.

Furthermore, the terms used herein are set taking into consideration the functions in the present invention and may vary depending on the intention of producers or usual practices, and the definitions thereof have to be determined based on the contents disclosed in the present specification.

Also, the sizes and thicknesses of the respective components shown in the drawings are arbitrarily shown for convenience of explanation, and thus the present invention is not limited to what is shown in the drawings.

In the present invention, in order to increase the yield of collagen at the time of sterilizing animal-derived tissue through irradiation with radioactive rays without collagen denaturation, animal-derived tissue is first irradiated with radioactive rays and thus sterilized.

Thereafter, the sterilized animal-derived tissue is washed and pulverized.

The animal-derived tissue irradiated with radioactive rays is preferably washed with distilled water and ethanol for 2 ~ 6 hr, thus removing impurities on the outside of the material. Specifically, distilled water is used to wash other impurities, including blood, microorganisms, and the like, and ethanol is used to wash fat.

Subsequently, the pulverized tissue is reacted with an acid solution or an enzyme solution, thus separating collagen.

Thereafter, a salt is added to thus extract collagen.

Subsequently, the salt is removed from the separated collagen, thereby increasing the yield of collagen.

In particular, the animal-derived tissue used in the present invention includes any one selected from among mammals, birds, and fish.

The radioactive rays preferably include gamma rays or electromagnetic rays.

Here, the radioactive rays are applied in a dose of 5 kGy ~ 40 kGy.

If the dose of radioactive rays is less than 5 kGy, there is no sterilization effect. On the other hand, if the dose thereof exceeds 40 kGy, tissue, especially collagen, may be denatured. Hence, the radioactive rays are applied in the dose range of 5 kGy ~ 40 kGy.

The dose (dose rate × time) of radioactive rays is preferably adjusted based on 2.7 ~ 3.7 kGy/hr, which is the dose rate of radioactive rays.

The particle size of the pulverized tissue is preferably 0.1 ~ 5.0 mm.

As such, in order to make the particle size less than 0.1 mm, the pulverization process has to be performed for a long period of time, and also a large amount of heat may be generated, and moreover, collagen denaturation may result. Hence, the particle size of the pulverized tissue is preferably set to 0.1 mm or more. If the particle size thereof exceeds 5.0 mm, the portion of tissue that is exposed in order to degrade tissue, using an enzyme or an acid, is reduced (i.e. the surface area thereof is decreased), and thus the separation yield of collagen may decrease. Hence, the particle size of the pulverized tissue is preferably set to 5.0 mm or less.

The particles of the pulverized tissue are preferably mixed with an acid solution (pH 1.5 ~ 3.5) in an amount 50 times the weight of the pulverized tissue and stirred for 3 ~ 7 days.

The reason why the acid solution is added in an amount 50 times the weight of the pulverized tissue is that collagen may be separated at the most efficient yield from the tissue, and the reason why the pH is 1.5 ~ 3.5 is that an enzyme that is active under acidic conditions is used. Furthermore, the tissue is softened by the acid solution, and collagen may be separated therefrom. The reason why the stirring time is 3 ~ 7 days is as follows. If the stirring time is less than 3 days, the collagen yield may decrease. On the other hand, if the stirring time exceeds 7 days, the yield is not increased. Hence, the optimal separation yield of collagen may be obtained for the above stirring time.

The acid solution preferably includes any one selected from among phosphoric acid, hydrochloric acid, citric acid, formic acid, and acetic acid aqueous solutions.

The enzyme solution preferably includes any one selected from among pepsin, papain, and trypsin aqueous solutions.

The final concentration of the added salt is preferably 0.6 ~ 1.0 M.

The salt concentration for selectively separating collagen is a salt concentration for selectively separating proteins corresponding to the molecular weight (100 ~ 300 kDa) of collagen.

Meanwhile, the present invention may be variously modified in various ways in applying the afore-mentioned construction.

The effects of performing the method of increasing the yield of collagen according to the present invention and collagen prepared thereby are described below.

In the present invention, animal-derived tissue is irradiated with radioactive rays to thus remove microorganisms and endotoxins therefrom, thereby increasing preservability and the yield of collagen without collagen denaturation.

In order to alleviate the problems with conventional methods to thereby simultaneously realize the sterilization of animal-derived tissue without collagen denaturation and an increase in the yield of collagen, the present invention pertains to a method of increasing the yield of collagen at the time of sterilizing animal-derived tissue using radioactive rays, which are economical and environmentally friendly and have high industrial applicability.

In the present invention, collagen is referred to a protein extracted from tissue of various animals through treatment with an acid or alkali or treatment with an enzyme such as pepsin, etc.

Below is a description of an embodiment of the present invention with reference to the appended drawings.

FIG. 1 shows the process of separating collagen from animal-derived tissue sterilized through irradiation with radioactive rays according to an embodiment of the present invention. With reference thereto, the method of obtaining collagen irradiated with radioactive rays is as follows.

In the present invention, collagen is prepared by:

1) sterilizing animal-derived tissue through irradiation with radioactive rays;
2) washing and pulverizing the sterilized animal-derived tissue;
3) separating collagen by reacting the pulverized tissue with an acid solution or an enzyme solution;
4) extracting collagen through addition of a salt; and
5) removing the salt from the separated collagen.

The method of the present invention is stepwisely specified below.

First, animal-derived tissue is irradiated with radioactive rays of 5 ~ 40 kGy, and preferably 5 ~ 25 kGy. The dose (dose rate × time) of radioactive rays is adjusted based on 2.7 ~ 3.7 kGy/hr, which is the dose rate of radioactive rays.

Examples of the animal-derived tissue that is used herein may include various tissues of mammals, birds, fish, etc.

The animal-derived tissue irradiated with radioactive rays is washed with distilled water and 70% ethanol for 2 ~ 6 hr, and preferably 3 ~ 5 hr.

The washed animal-derived tissue is pulverized to a size of 0.1 ~ 5.0 mm, and preferably 0.5 ~ 3.0 mm.

The pulverized tissue is mixed with an acid solution (pH 1.5 ~ 3.5) in an amount 50 times the weight thereof and stirred for 3 ~ 7 days and is preferably mixed with an acid solution at a pH of 2.0 ~ 2.5 and stirred for 4 ~ 5 days.

Here, the acid solution may be prepared using phosphoric acid, hydrochloric acid, citric acid, formic acid, or acetic acid, and depending on the type of separation of collagen, an alkali solution or a protease (pepsin, papain, or trypsin) may be used.

In order to extract collagen, NaCl is added so that the final salt concentration is 0.6 ~ 1.0 M, and preferably 0.7 ~ 0.9 M.

In order to remove the salt from the extracted collagen, distilled water is added in an amount 5 ~ 20 times the weight of collagen and ultrafiltration is then conducted, and preferably, distilled water is added in an amount 10 ~ 15 times the weight of collagen and ultrafiltration is then conducted.

As an alternative to the above process in order to remove the salt, centrifugation after neutralization of the collagen solution may be performed.

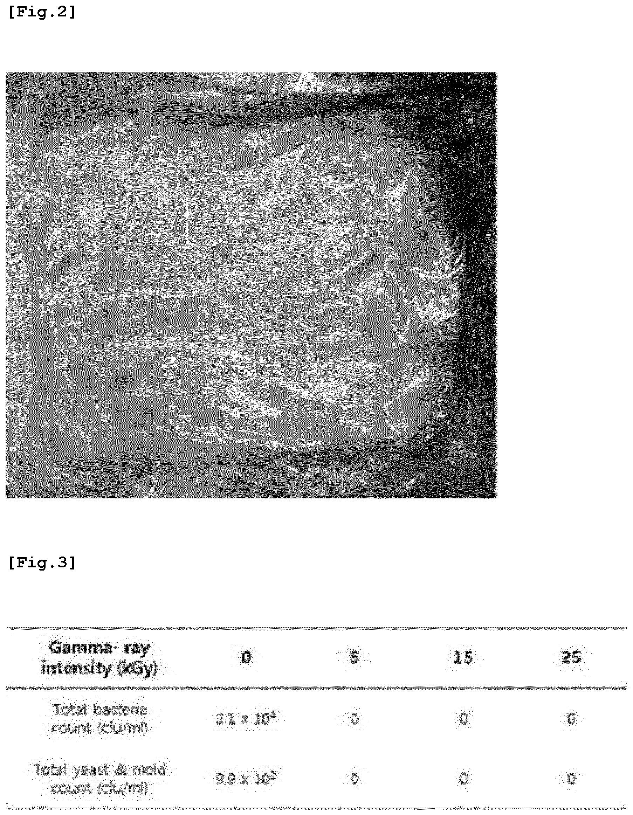

FIG. 2 is a photograph showing the animal-derived tissue irradiated with gamma rays after sealing in order to realize long-term preservation according to the present invention.

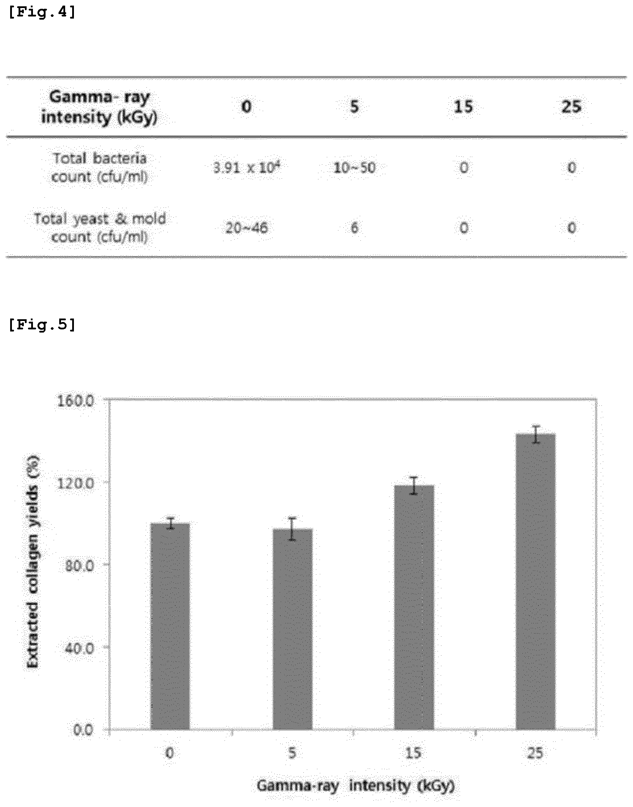

FIG. 3 shows the extent of sterilization of bacteria and fungi after irradiation of pig skin tissue with gamma rays according to the present invention, and FIG. 4 shows the extent of sterilization of bacteria and fungi after irradiation of skin tissue of duck's feet with gamma rays according to the present invention.

Thereby, the animal-derived tissue sterilized through irradiation with radioactive rays may be obtained, which is described in the following [Example 1].

[Example 1]

Pig skin tissue or tissue of duck's feet, washed with distilled water, was irradiated with gamma rays of 0, 5, 15, and 25 kGy.

After completion of irradiation with gamma rays, 1 g of the tissue specimen was added with 1 mL of an isotonic buffer solution, mixed with stirring, and then applied in an amount of 1 mL onto bacterial and fungal culture solid media.

Bacteria were incubated at 30 ~ 35°C for 2 days to thus measure the total bacterial count, and fungi were incubated at 20 ~ 25°C for 5 days to thus measure the total fungal count.

In the case of pig skin tissue, upon irradiation with gamma rays of 5 kGy or more, all of bacteria and fungi were confirmed to be killed, and in the case of tissue of duck's feet, upon irradiation with gamma rays of 15 kGy or more, all of bacteria and fungi were confirmed to be killed.

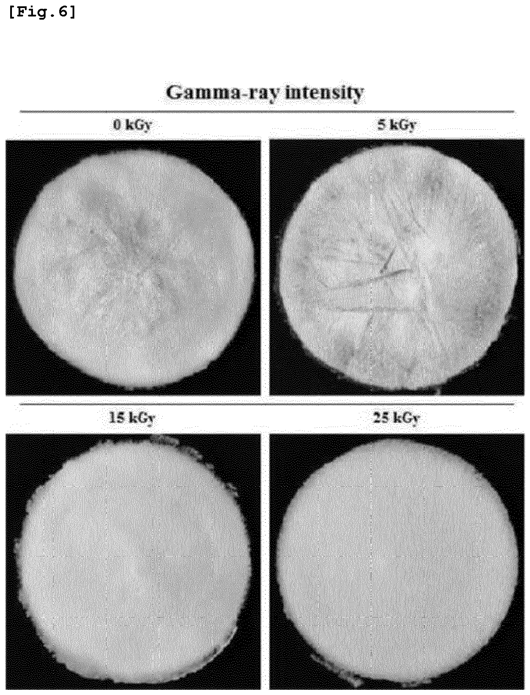

FIG. 5 is a graph showing the yield of collagen separated through acid treatment with gamma rays applied in different amounts according to the present invention, and FIG. 6 is of photographs showing collagen lyophilized after separation through acid treatment with gamma rays applied in different amounts according to the present invention.

Collagen may be separated from the animal-derived tissue through the above preparation process, which is described in the following [Example 2].

[Example 2]

The tissue of duck's feet, washed with distilled water, was irradiated with gamma rays of 0, 5, 15, and 25 kGy.

The tissue of duck's feet, irradiated with radioactive rays, was washed in a manner in which it was washed three times with distilled water, immersed in 70% ethanol in an amount at least 5 times the weight thereof and stirred.

The washed tissue of duck's feet was pulverized into a size of 0.5 ~ 3.0 mm, and the pulverized tissue was mixed with an acid solution (pH 2.0) in an amount 50 times the weight thereof and stirred for 5 days.

After 5 days, in order to extract collagen from the acid solution in which the tissue of duck's feet was degraded, NaCl was added such that the final salt concentration was 0.9 M.

The extracted collagen was dissolved again in distilled water in an amount 5 times the volume thereof, thus preparing a collagen solution.

In order to remove the salt from the collagen solution, the pH of the solution was adjusted to 7.0, followed by centrifugation, thus obtaining collagen.

The yield (%) of the obtained collagen was calculated using the following equation.

$$Y_x = \frac{weight\ of\ extracted\ collagen\ (x kGy)}{weight\ of\ extracted\ collagen\ (0 kGy)} \times 100$$

Y:    yield of obtained collagen (%)
x:    dose of radioactive rays (kGy)

Consequently, the yield of collagen separated from the tissue of duck's feet irradiated with gamma rays of 15 kGy was 118.313.9%, and the yield of collagen separated from the tissue of duck's feet irradiated with gamma rays of 25 kGy was 143.213.8%.

As shown in FIG. 6, based on the results of lyophilization of the separated collagen, the separation yield was significantly increased to such an extent that it could be distinguished by the naked eye.

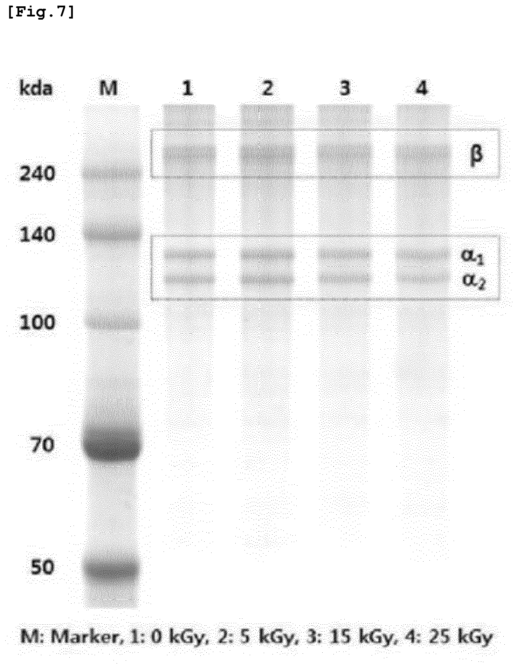

FIG. 7 is a photograph showing the results of SDS-PAGE of collagen separated through acid treatment with gamma rays applied in different amounts according to the present invention.

The collagen separated from the animal-derived tissue through the above preparation process was evaluated for denaturation of protein molecules through SDS-PAGE, which is described in the following [Example 3].

[Example 3]

The collagen separated from the tissue of duck's feet irradiated with gamma rays of 0, 5, 15, and 25 kGy was diluted to 10 mg/ml using distilled water.

The diluted collagen solution was mixed with 5x sample-loading buffer (0.3 M Tris-HCl (pH 6.8), 0.1% bromophenol blue, 10.0% SDS, 50.0% glycerol, and 12.5% β-mercaptoethanol) and then heated at 100°C for 3 min.

The prepared sample was loaded in an amount of 15 μL per well of a 10.0% polyacrylamide gel and then subjected to electrophoresis at 100 V for 110 min.

After completion of electrophoresis, the gel was stained with 0.3% (w/v) Coomassie Brilliant Blue-R250, and the background thereof was decolorized with a destaining reagent (50.0% distilled water, 40.0% methanol, 10.0% acetic acid), followed by analysis.

As illustrated in FIG. 7, in all samples irradiated with gamma rays, $\alpha_1$, $\alpha_2$ and $\beta$ chains, corresponding to the properties of Type I collagen, were observed, and collagen did not denature upon irradiation with gamma rays in the dose range of 0 ~ 25 kGy.

**Industrial Applicability**

The technical idea of the present invention regarding the method of increasing the yield of collagen and the collagen prepared thereby is able to obtain consistent results in practice. In particular, the present invention promotes technical development and can contribute to industrial development and is thus worth protecting.

**Claims**

1. A method of increasing a collagen yield, suitable for sterilizing animal-derived tissue through irradiation with radioactive rays without collagen denaturation and simultaneously increasing a yield of collagen, the method comprising the steps in the following order:

   sterilizing animal-derived tissue through irradiation with radioactive rays in a dose of 5 kGy ~ 40 kGy;
   washing and pulverizing the sterilized animal-derived tissue;
   separating collagen by reacting the pulverized tissue with an acid solution or an enzyme solution, the stirring time being 3 ~ 7 days in case an acid solution is used;
   extracting the collagen through addition of a salt; and
   removing the salt from the separated collagen.

2. The method of claim 1, wherein the animal-derived tissue includes any one selected from among a mammal, a bird, and a fish.

3. The method of claim 1, wherein the radioactive rays are gamma rays or electromagnetic rays.

4. The method of claim 1, wherein the dose (dose rate × time) of the radioactive rays is adjusted based on 2.7 ~ 3.7 kGy/hr, which is a dose rate of the radioactive rays.

5. The method of claim 1, wherein the pulverized tissue has a particle size of 0.1 ~ 5.0 mm.

6. The method of claim 1 or 5, wherein particles of the pulverized tissue are mixed with an acid solution (pH 1.5 ~ 3.5) in an amount 50 times a weight of the pulverized tissue, and stirred for 3 ~ 7 days.

7. The method of claim 1, wherein the acid solution includes any one selected from among phosphoric acid, hydrochloric acid, citric acid, formic acid, and acetic acid aqueous solutions.

8. The method of claim 1, wherein the enzyme solution includes any one selected from among pepsin, papain, and trypsin aqueous solutions.

9. The method of claim 1, wherein a final concentration of the added salt is 0.6 ~ 1.0 M.

10. The method of claim 1, wherein the animal-derived tissue irradiated with radioactive rays is washed with distilled water and ethanol for 2 ~ 6 hr.

11. The method of claim 1, wherein the step of removing the salt is performed through ultrafiltration or through centrifugation after neutralization.

**Patentansprüche**

1. Verfahren zur Erhöhung einer Collagenausbeute, das zum Sterilisieren von tierischem Gewebe durch Bestrahlung mit radioaktiven Strahlen ohne Collagendenaturierung und gleichzeitige Erhöhung einer Ausbeute von Collagen geeignet ist, wobei das Verfahren die Schritte in der folgenden Reihenfolge umfasst:

   Sterilisieren von tierischem Gewebe durch Bestrahlung mit radioaktiven Strahlen in einer Dosis von 5 kGy bis 40 kGy;

Waschen und Pulverisieren des sterilisierten tierischen Gewebes;
Abtrennen von Collagen durch Umsetzen des pulverisierten Gewebes mit einer Säurelösung oder einer Enzymlösung, wobei die Rührzeit 3~7 Tage beträgt, falls eine Säurelösung verwendet wird;
Extrahieren des Collagens durch Addition eines Salzes; und
Entfernen des Salzes aus dem abgetrennten Collagen.

2.  Verfahren gemäß Anspruch 1, wobei das tierische Gewebe eines umfasst, das aus einem Gewebe ausgewählt ist, das von einem Säuger, Vogel oder Fisch stammt.

3.  Verfahren gemäß Anspruch 1, wobei die radioaktiven Strahlen Gammastrahlen oder elektromagnetische Strahlen sind.

4.  Verfahren gemäß Anspruch 1, wobei die Dosis (Dosisrate x Zeit) der radioaktiven Strahlen auf der Basis von 2,7~3,7 kGy/h, der Dosisrate der radioaktiven Strahlen, eingestellt wird.

5.  Verfahren gemäß Anspruch 1, wobei das pulverisierte Gewebe eine Teilchengröße von 0,1~5,0 mm aufweist.

6.  Verfahren gemäß Anspruch 1 oder 5, wobei Teilchen des pulverisierten Gewebes mit einer Säurelösung (pH 1,5~3,5) in einer Menge, die dem 50-fachen Gewicht des pulverisierten Gewebes entspricht, gemischt und 3~7 Tage lang gerührt werden.

7.  Verfahren gemäß Anspruch 1, wobei die Säurelösung eine umfasst, die aus wässrigen Phosphorsäure-, Chlorwasserstoffsäure-, Zitronensäure-, Ameisensäure- und Essigsäurelösungen ausgewählt ist.

8.  Verfahren gemäß Anspruch 1, wobei die Enzymlösung eine umfasst, die aus wässrigen Pepsin-, Papain- und Trypsinlösungen ausgewählt ist.

9.  Verfahren gemäß Anspruch 1, wobei die endgültige Konzentration des hinzugefügten Salzes 0,61,0 M beträgt.

10. Verfahren gemäß Anspruch 1, wobei das mit radioaktiven Strahlen bestrahlte tierische Gewebe 2~6 Stunden lang mit destilliertem Wasser und Ethanol gewaschen wird.

11. Verfahren gemäß Anspruch 1, wobei der Schritt des Entfernens des Salzes nach Neutralisation durch Ultrafiltration oder durch Zentrifugation durchgeführt wird.

## Revendications

1.  Procédé pour augmenter un rendement de collagène, approprié pour la stérilisation d'un tissu provenant d'un animal par irradiation avec des rayons radioactifs sans dénaturation du collagène tout en augmentant un rendement de collagène, le procédé comprenant les étapes suivantes dans l'ordre suivant, consistant à :

    stériliser un tissu provenant d'un animal par irradiation avec des rayons radioactifs à une dose de 5 kGy~40 kGy,
    laver et pulvériser le tissu provenant d'un animal, stérilisé,
    séparer le collagène en faisant réagir le tissu pulvérisé avec une solution d'acide ou une solution d'enzyme, le temps d'agitation étant 3~7 jours dans le cas où une solution d'acide est utilisée,
    extraire le collagène par l'ajout d'un sel, et
    éliminer le sel du collagène séparé.

2.  Procédé selon la revendication 1, dans lequel ledit tissu provenant d'un animal comprend l'un quelconque choisi parmi un mammifère, un oiseau et un poisson.

3.  Procédé selon la revendication 1, dans lequel lesdits rayons radioactifs sont des rayons gamma ou des rayonnements électromagnétiques.

4.  Procédé selon la revendication 1, dans lequel la dose (débit de dose x temps) desdits rayons radioactifs est ajustée sur la base de 2,7~3,7 kGy/h, qui est le débit de dose des rayons radioactifs.

5. Procédé selon la revendication 1, dans lequel le tissu pulvérisé présente une granulométrie de 0,1~5,0 mm.

6. Procédé selon la revendication 1 ou 5, dans lequel des particules du tissu pulvérisé sont mélangées avec une solution d'acide (pH 1,5~3,5) en une quantité correspondant à 50 fois le poids du tissu pulvérisé, et agitées pendant 3~7 jours.

7. Procédé selon la revendication 1, dans lequel la solution d'acide comprend l'une quelconque choisie parmi les solutions aqueuses d'acide phosphorique, d'acide chlorhydrique, d'acide citrique, d'acide formique, et d'acide acétique.

8. Procédé selon la revendication 1, dans lequel la solution d'enzyme comprend l'une quelconque choisie parmi les solutions aqueuses de pepsine, papaïne et trypsine.

9. Procédé selon la revendication 1, dans lequel la concentration finale du sel ajouté est de 0,6~1,0 M.

10. Procédé selon la revendication 1, dans lequel le tissu provenant d'un animal, irradié avec des rayons radioactifs, est lavé avec de l'eau distillée et de l'éthanol pendant 2~6 heures.

11. Procédé selon la revendication 1, dans lequel l'étape consistant à éliminer le sel est effectuée par ultrafiltration ou par centrifugation après neutralisation.

[Fig.1]

```
┌─────────────────────────────────┐
│  Irradiation of animal-derived  │
│    tissue with radioactive rays │
└─────────────────────────────────┘
                 │
                 ▼
┌─────────────────────────────────┐
│ Washing and pulverization of    │
│            tissue               │
└─────────────────────────────────┘
                 │
                 ▼
┌─────────────────────────────────┐
│  Treatment with acid solution or│
│  enzyme solution                │
└─────────────────────────────────┘
                 │
                 ▼
┌─────────────────────────────────┐
│   Extraction of collagen salt   │
└─────────────────────────────────┘
                 │
                 ▼
┌─────────────────────────────────┐
│        Removal of salt          │
└─────────────────────────────────┘
```

[Fig.2]

[Fig.3]

| Gamma- ray intensity (kGy) | 0 | 5 | 15 | 25 |
|---|---|---|---|---|
| Total bacteria count (cfu/ml) | $2.1 \times 10^4$ | 0 | 0 | 0 |
| Total yeast & mold count (cfu/ml) | $9.9 \times 10^2$ | 0 | 0 | 0 |

[Fig.4]

| Gamma-ray intensity (kGy) | 0 | 5 | 15 | 25 |
|---|---|---|---|---|
| Total bacteria count (cfu/ml) | $3.91 \times 10^4$ | 10~50 | 0 | 0 |
| Total yeast & mold count (cfu/ml) | 20~46 | 6 | 0 | 0 |

[Fig.5]

[Fig.6]

Gamma-ray intensity

[Fig.7]

M: Marker, 1: 0 kGy, 2: 5 kGy, 3: 15 kGy, 4: 25 kGy

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 0837858
- KR 20120084189
- KR 20140118763
- WO 2011014155 A
- WO 2006096027 A
- WO 2006124988 A
- US 20160052962 A